(19)

Europäisches
Patentamt
European
Patent Office
Office européen
des brevets

(11) **EP 4 382 598 A1**

(12) **EUROPEAN PATENT APPLICATION**
published in accordance with Art. 153(4) EPC

(43) Date of publication:
**12.06.2024   Bulletin 2024/24**

(21) Application number: **22853015.0**

(22) Date of filing: **01.08.2022**

(51) International Patent Classification (IPC):
*C12N 5/074* (2010.01)    *C12N 5/071* (2010.01)
*C12N 5/0775* (2010.01)    *C12N 5/10* (2006.01)

(52) Cooperative Patent Classification (CPC):
**C12N 5/06; C12N 5/10**

(86) International application number:
**PCT/JP2022/029522**

(87) International publication number:
**WO 2023/013596 (09.02.2023 Gazette 2023/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:   **02.08.2021   JP 2021126706**

(71) Applicant: **Rainbow Inc.
Sapporo-shi, Hokkaido, 001-0021 (JP)**

(72) Inventor: **KAWABORI, Masahito
Sapporo-shi, Hokkaido 060-0808 (JP)**

(74) Representative: **Vossius & Partner
Patentanwälte Rechtsanwälte mbB
Siebertstraße 3
81675 München (DE)**

Remarks:
The complete document including Reference
Table(s) and the Sequence Listing(s) can be
downloaded from the EPO website

(54)    **METHOD FOR STORING OR TRANSPORTING STEM CELLS IN UNFROZEN STATE**

(57)    The present disclosure provides a method of storing stem cells while maintaining high viability at low temperatures in an unfrozen state. In one aspect, the present disclosure is a method for storing stem cells and provides the method including: 1) a step of providing a storage solution including a gelling agent at a temperature more than a gelling temperature in a non-gelled state and having a pH of about 5.0 to about 8.0; 2) a step of putting the stem cells into the storage solution and then lowering a temperature to the gelling temperature of the gelling agent; and 3) a step of storing the storage solution including the stem cells at a gel-state maintaining temperature of the gelling agent. In some embodiments, the gelling agent may include collagen, denatured collagen, collagen-like peptide, gelatin, or any combination thereof.

[Fig. 1]

Fig. 1   VIABILITY AFTER STORAGE FOR 72 HOURS

VIABILITY IN 72 HOURS AFTER RESEEDING

**Description**

Technical Field

The present disclosure relates to a technique for storing or transporting stem cells.

Background Art

Currently, regenerative medicine products with a plurality of stem cells have been developed and partially approved in Japan, and these have brought new hope to diseases for which there has been no treatment (severe burns, spinal cord injury, head injury, cerebral infarction, cerebral hemorrhage, Parkinson's disease, and the like), and great development is expected in the future. However, many of these products are transported in a cryostorage state at ultra-low temperature (-170°C). This has problems of (1) a decrease in cell quality (cell viability and nutrient factor secretion) and (2) transportation cost (liquid nitrogen cost and purchase and maintenance of ultra-low temperature transportation equipment). On the other hand, when cells are transported at normal temperature, there are such a problem that oxygen and nutrients in the transport liquid are depleted because cell metabolism proceeds as usual, and such a problem that physical damage due to vibration occurs because the cells are not completely fixed, and the viability for a long time (one day or more) is very low. In the case of transportation at a low temperature, metabolism is suppressed, but vibration is not improved. There is a need for a method allowing inexpensive transportation that can withstand transportation conditions such as vibration while maintaining cell viability.

Summary of Invention

Solution to Problem

As a result of diligent efforts, the present disclosures have found a method for storing stem cells at a high viability. Specifically, the present disclosures have found that stem cells are stored with a gelling agent to maintain high viability and the ability to secrete nutritional factors in an unfrozen state. Thus, according to the storage method according to the present disclosure, it is possible to transport therapeutic stem cells at low temperature (for example, 4°C) in an unfrozen state.

Accordingly, the present disclosure provides the following.

(Clause 1) A method for storing stem cells, the method including:

1) a step of providing a storage solution including a gelling agent at a temperature more than a gelling temperature and having a pH of about 5.0 to about 8.0;
2) a step of putting the stem cells into the storage solution and then lowering a temperature to the gelling temperature of the gelling agent; and
3) a step of storing the storage solution including the stem cells at a gel-state maintaining temperature of the gelling agent.

(Clause 1A) A method for storing stem cells, the method including:

1) a step of providing a storage solution including a gelling agent at a temperature more than a gelling temperature in a non-gelled state and having a pH of about 5.0 to about 8.0;
2) a step of putting the stem cells into the storage solution and then lowering a temperature to the gelling temperature of the gelling agent; and
3) a step of storing the storage solution including the stem cells at a gel-state maintaining temperature of the gelling agent.

(Clause 2)
The method according to the above clause, wherein the gelling agent includes a peptide.
(Clause 3)
The method according to any one of the above clauses, wherein the gelling agent includes collagen, denatured collagen, collagen-like peptide, gelatin or any combination thereof.
(Clause 4)
The method according to any one of the above clauses, wherein the gelling agent includes a collagen-like peptide, the collagen-like peptide includes a repeat of a sequence represented by Gly-X-Y, and X and Y are each independently identical or different amino acids.

(Clause 5)

The method according to any one of the above clauses, wherein the gelling agent is included in the storage solution at a concentration of about 1% (w/w) or more.

(Clause 6)

The method according to any one of the above clauses, wherein the gelling agent is included in the storage solution at a concentration of about 1% (w/w) to about 10% (w/w).

(Clause 7)

The method according to any one of the above clauses, wherein the gelling agent is included in the storage solution at a concentration of about 2.5% (w/w).

(Clause 8)

The method according to any one of the above clauses, wherein the storage solution is a cell culture solution, physiological saline, or an electrolyte solution.

(Clause 9)

The method according to any one of the above clauses, wherein the pH of the storage solution is about 6.0 to about 7.5.

(Clause 10)

The method according to any one of the above clauses, wherein the pH of the storage solution is about 6.4 to about 7.4.

(Clause 11)

The method according to any one of the above clauses, wherein the gel-state maintaining temperature is about 0°C to about 37°C.

(Clause 12)

The method according to any one of the above clauses, wherein the gel-state maintaining temperature is about 0°C to about 24°C.

(Clause 13)

The method according to any one of the above clauses, wherein the stem cells are mesenchymal stem cells.

(Clause 14)

The method according to any one of the above clauses, wherein the mesenchymal stem cells are mesenchymal stem cell stem cells derived from bone marrow, adipose tissue, placental tissue, synovial tissue, umbilical cord tissue (for example, cord blood), dental pulp, or amniotic membrane, or mesenchymal stem cells differentiated from ES cells or iPS cells.

(Clause 15)

The method according to any one of the above clauses, wherein the mesenchymal stem cells are mesenchymal stem cells derived from bone marrow.

(Clause 16)

The method according to any one of the above clauses, wherein the stem cells are cells used in cell infusion therapy and drug discovery research and are obtained from a subject receiving the cell infusion therapy.

(Clause 17)

The method according to any one of the above clauses, further including a step of non-gelling treatment.

(Clause 18)

The method according to any one of the above clauses, wherein the step of non-gelling treatment includes increasing a temperature to a melting point of the gelling agent or a temperature higher than the melting point.

(Clause 19)

The method according to any one of the above clauses, further including a step of collecting the stem cells.

(Clause 20)

The method according to any one of the above clauses, wherein the step of collecting the stem cells includes diluting the storage solution 2-fold to 20-fold and separating the stem cells and the storage solution.

(Clause 21)

The method according to any one of the above clauses, wherein the stem cells are stored at a cell density of about $1 \times 10^6$ to about $1 \times 10^7$ cells/ml.

(Clause 22)

A kit for storing stem cells, the kit including:

(1) a storage solution including a gelling agent; and
(2) a buffer solution for adjusting a pH of the storage solution to about 5.0 to about 8.0, wherein the stem cells are stored at a gel-state maintaining temperature.

(Clause 23)

A composition for storing stem cells, the composition including a gelling agent.

(Clause 24)

The composition according to any one of the above clauses, wherein the composition is used at a pH of about 5.0 to about 8.0 in storing stem cells.

(Clause 25)

The composition according to any one of the above clauses, wherein the composition is used to store stem cells at a gelling temperature of the gelling agent.

(Clause 26)

A formulation including a gelling agent and stem cells, wherein a pH is about 5.0 to about 8.0.

(Clause 29)

The formulation according to any one of the above clauses, wherein the formulation is a Ready-to-Use formulation.

(Clause 30)

A method for storing a tissue or organ, the method including:

1) a step of providing a storage solution including a gelling agent at a temperature more than a gelling temperature and having a pH of about 5.0 to about 8.0;

2) a step of putting the tissue or organ into the storage solution and then lowering a temperature to the gelling temperature of the gelling agent; and

3) a step of storing the storage solution including the tissue or organ at a gel-state maintaining temperature of the gelling agent.

(Clause 30A)

A method for storing a tissue or organ, the method including:

1) a step of providing a storage solution including a gelling agent at a temperature more than a gelling temperature in a non-gelled state and having a pH of about 5.0 to about 8.0;

2) a step of putting the tissue or organ into the storage solution and then lowering a temperature to the gelling temperature of the gelling agent; and

3) a step of storing the storage solution including the tissue or organ at a gel-state maintaining temperature of the gelling agent.

(Clause 31)

A kit for storing a tissue or organ, the kit including:

(1) a storage solution including a gelling agent; and

(2) a buffer solution for adjusting a pH of the storage solution to about 5.0 to about 8.0, wherein the tissue or organ are stored at a gel-state maintaining temperature.

(Clause 32)

A composition for storing a tissue or organ, the composition including a gelling agent.

(Clause 33)

The composition according to any one of the above clauses, wherein the composition is used at a pH of about 5.0 to about 8.0 in storing a tissue or organ.

(Clause 34)

A method for conveying stem cells, the method including:

1) a step of providing a storage solution including a gelling agent at a temperature more than a gelling temperature and having a pH of about 5.0 to about 8.0;

2) a step of putting the stem cells into the storage solution and then lowering a temperature to the gelling temperature of the gelling agent; and

3) a step of conveying the storage solution including the stem cells at a gel-state maintaining temperature of the gelling agent.

(Clause 33)

A gelling agent for storing stem cells, wherein the gelling agent is used at a gel-state maintaining temperature of the gelling agent at a pH of about 5.0 to about 8.0 in storing the stem cells.

(Clause 34)

A gelling agent for storing a tissue or organ, wherein the gelling agent is used at a gel-state maintaining temperature of the gelling agent at a pH of about 5.0 to about 8.0 in storing the tissue or organ.

In the present disclosure, it is intended that one feature or a plurality of features described above may be provided in further combination in addition to the specified combination. A still further embodiment and advantage of the present disclosure will be appreciated by those skilled in the art upon reading and understanding the following detailed description as appropriate.

Brief Description of Drawings

FIG. 1 shows graphs of cell viability after storage for 72 hours and in 72 hours after reseeding in the presence or absence of RCP.
FIG. 2 shows graphs of cell viability after storage for 72 hours and in 72 hours after reseeding in the presence of different concentrations of RCP.
FIG. 3 shows graphs of cell viability after storage for 72 hours and in 72 hours after reseeding in different storage solutions containing RCP.
FIG. 4 shows graphs of cell viability after storage for 72 hours and in 72 hours after reseeding in storing at different temperatures.
FIG. 5 shows graphs of cell viability after storage for 72 hours and in 72 hours after reseeding in storing in different storing days.
FIG. 6 shows graphs of cell viability after storage for 72 hours and in 72 hours after reseeding in storage solutions containing different gelling agents.
FIG. 7 shows graphs of cell viability after storage for 72 hours and in 72 hours after reseeding in storing at different pH values.
FIG. 8 shows graphs of cell viability after transporting cells and cell viability after cells are left to stand and stored.
FIG. 9 is graphs showing cell viability when stored cells are diluted 1-fold and 5-fold with a storage solution.

Description of Embodiments

Hereinafter, the present disclosure will be described. It is to be understood that throughout the description, the singular forms of expression also include the concept of the plural forms thereof, unless otherwise stated. Thus, it is to be understood that the singular article (for example, in English, "a", "an", "the", and the like are used) also includes the plural concept thereof unless otherwise stated. It is also to be understood that the terms used in the present description are used in the sense commonly used in the art unless otherwise noted. Thus, unless defined otherwise, all technical and scientific terms used in the present description have the same meaning as commonly understood by one of ordinary skill in the art to which this disclosure belongs. In case of conflict, the present description (including definitions) will control.

(Definitions)

As used in the present description, "about" means ± 10% of the value that follows.
In the present description, the "stem cell" means an immature cell having an ability of self-replication and abilities of differentiation and proliferation. Stem cells include subpopulations such as pluripotent stem cells, multipotent stem cells, and unipotent stem cells, depending on differentiation capacity. The pluripotent stem cell means a cell that cannot become an individual by itself but has the ability to differentiate into all tissues and cells constituting a living body. The pluripotent stem cell means a cell having the ability to differentiate into a plurality of types of tissues and cells, although not all types. The unipotent stem cell means a cell having the ability to differentiate into a specific tissue or cell.
The term "mesenchymal stem cell" in the present description is also referred to as a mesenchymal stromal cell, and refers to a stem cell having the ability to differentiate into a cell belonging to the mesenchymal system, such as an osteoblast, an adipocyte, a myocyte, or a chondrocyte. The mesenchymal stem cell may include a mesenchymal stem cell stem cell derived from bone marrow, adipose tissue, placental tissue, synovial tissue, umbilical cord tissue (for example, cord blood), dental pulp, or amniotic membrane, or a mesenchymal stem cell differentiated from an ES cell or iPS cell.
In the present description, "tissue" refers to any tissue type, including any type of cell type and combinations thereof, such as ovarian tissue, testicular tissue, umbilical cord tissue, placental tissue, connective tissue, cardiac tissue, corneal tissue, muscle, cartilage or bone derived tissue, endocrine tissue, and neural tissue.
In the present description, an "organ" refers to a lung, a liver, a kidney, a heart, an ovary, a pancreas, and an umbilical cord. The organ may be a human organ or a non-human animal organ. The non-human animal may be a rodent including a mouse and a rat, an ungulate including a pig, a goat, and a sheep, a non-human primate including a chimpanzee, another non-human mammal, or an animal other than a mammal.

In the present description, "storage" means storing in a container for a certain period of time for any purpose (for example, cell infusion therapy, tissue or organ transplantation or transport therefor), and refers to maintaining cells in the container while maintaining the function of cells, a tissue(s), or an organ(s) without the purpose of growing the cells. Storing is different from "culture", which is intended to grow cells. In addition, the storing of cells does not mean that the cells are transferred into a container such as a syringe immediately before administration and temporarily retained, or that the cells are temporarily retained in the container for preparation at time of use before administration.

In the present description, "storage solution" refers to a solution for maintaining cells for a certain period of time, and refers to a solution having a physiological osmotic pressure and an electrolyte to the extent that cells can survive.

In the present description, "gelling agent" refers to a drug for gelling a storage solution. As the gelling agent used in the present description, there may be advantageously used a gelling agent having a property of not being gelled in a normal environment (for example, at a temperature higher than a gelling temperature (for example, when the gelling temperature is 24°C or less, the temperature may be 37°C or the like)) when being contained in a storage solution, and being gelled at a gelling temperature.

In the present description, the "gelling temperature" refers to a temperature at which a non-gelled state changes to a gelled state.

In the present description, the "gel-state maintaining temperature" refers to a temperature at which a gel is maintained without melting after gelation.

In the present description, "non-gelled" refers to removing the gelled state by heating, diluting with a solvent, or a combination thereof. The state in which the gelled state is removed is also referred to as a "non-gelled state". "Non-gelling temperature" refers to a temperature at which the gelled state changes to the non-gelled state. The non-gelling temperature is typically higher than the gelling temperature.

In the present description, "collagen" is a protein derived from an animal in which three polypeptide chains composed of amino acids have a triple helical structure, and there are 28 types of collagens confirmed in humans, and other collagens exist. When heated, collagen is thermally denatured, and the polypeptide chain is unraveled to form gelatin. There are two types of collagens: "denatured" and "non-denatured", which, when referred to, in the present description, simply as "collagen", is, unless otherwise stated, "non-denatured" collagen, which encompasses both "denatured" and "non-denatured", depending on the context. "Denatured" is applied with heat and is not recognized as collagen when absorbed into the body, whereas "non-denatured" is extracted without applying heat and thus is recognized as collagen in the body and absorbed as it is to be utilized as collagen in the body. The collagen is a type of gelling agents.

In the present description, "denatured collagen" refers to a collagen in which collagen is formed by thermal denaturation, chemical denaturation (for example, denaturation by collagenase), or denaturation due to mechanical damage, and some maintain the structure of collagen. The denatured collagen does not include a collagen in which collagen is completely denatured and changed into gelatin. Denatured collagen is a type of gelling agents.

In the present description, "gelatin" refers to a gelatin in which collagen is completely thermally denatured and has no triple helical structure unique to collagen. The gelatin is a type of gelling agents.

In the present description, "collagen-like peptide" refers to a chemically synthesized peptide designed to mimic the unique triple helical structure that characterizes collagen molecules. The collagen-like peptide is a type of gelling agents.

In the present description, "RCP" is an abbreviation for recombinant collagen peptide, and refers to a recombinantly expressed collagen-like peptide. The RCP is identical in structure to a collagen-like peptide and refers to a peptide recombinantly expressed. The RCP is a type of gelling agent.

In the present description, "cell infusion therapy" refers to a therapeutic method of infusing cells (for example, stem cells) for the treatment of a disease (for example, cerebral infarction).

In the present description, "Ready-to-Use formulation" refers to a formulation that can be used as it is without further culturing stored therapeutic cells.

(Preferable embodiments)

Although a description of preferable embodiments is described below, it is to be understood that this embodiment is illustrative of the disclosure and that the scope of the disclosure is not limited to such preferable embodiments. It should be understood that those skilled in the art can also easily make modifications, changes, and the like within the scope of the present disclosure with reference to the following preferable examples. For these embodiments, those skilled in the art may appropriately combine any embodiments.

(Method of storing stem cells)

In one aspect, the present disclosure is a method for storing stem cells and provides the method including: 1) a step of providing a storage solution including a gelling agent at a temperature more than a gelling temperature in a non-gelled state and having a pH of about 5.0 to about 8.0; 2) a step of putting the stem cells into the storage solution and then lowering a temperature to the gelling temperature of the gelling agent; and 3) a step of storing the storage solution including the stem cells at a gel-state maintaining temperature of the gelling agent. The storage method of the present disclosure can store stem cells while maintaining a high viability and the ability to secrete nutritional factors. It has been found that the viability is not reduced by vibration during storing, and the storage method of the present disclosure may also be applied to transportation in which vibration may occur.

In some embodiments, storing may involve conveying.

In embodiments, the gelling agent is any substance that causes a storage solution to gel, such as peptide, collagen, denatured collagen, collagen-like peptide, gelatin, fibrin, silicone, glycosaminoglycan, VitroGel™3D, Vitro-Gel™3D-RGB (TheWell Bioscience Inc.), BD Matrigel™ matrix (BD Bioscience Inc.), carboxyvinyl polymers, acrylic copolymers (for example, acrylate/alkyl acrylate copolymers), polyacrylamides, polysaccharides, natural gums, metal salts of fatty acids, hydrophobic silicas, polyethylene, crosslinked acrylic acid polymer (for example, carbomer, carboxypolyalkylene, Carbopol (registered trademark), polyethylene oxide, polyoxyethylene-polyoxypropylene copolymer, polyvinyl alcohol, cellulose-based polymer (for example, hydroxypropyl cellulose, hydroxyethyl cellulose, hydroxypropyl methyl cellulose, hydroxypropyl methyl cellulose phthalate, and methyl cellulose), gums (for example, gum tragacanth and xanthan gum), sodium alginate, gellan gum, agar, carrageenan, pectin, furcellaran, alginic acid or a salt thereof, glucomannan, tara gum, locust bean gum, tamarind, pullulan, guar gum, starch phosphate, polyacrylate, gum arabic, curdlan, ghatti gum, aeromonas gum, and tamarind seed polysaccharide, but not limited thereto.

In a certain embodiment, the gelling agent may be at least one selected from the group consisting of collagen, denatured collagen, collagen-like peptides, and gelatin.

In a certain embodiment, the gelling agent may be a collagen-like peptide. As the collagen-like peptide, commercially available products such as those provided by Nitta Gelatin Inc. (beMATRIX (registered trademark) collagen) or Fujifilm Corporation (recombinant peptide (RCP)) may be used. The collagen-like peptide includes repeats of the sequence represented by Gly-X-Y, and X and Y may be each independently the same amino acid or different amino acids.

In a further embodiment, the gelling agent may be a recombinant collagen peptide (RCP). In a certain embodiment, the RCP may consist of or include the amino acid sequence described in SEQ ID NO: 1, or an amino acid sequence having at least about 80%, about 85%, about 90%, about 95%, about 96%, about 97%, about 98%, or about 99% sequence identity thereto. The RCP may be prepared or purchased as appropriate according to WO 2018/159797 (Fujifilm Corporation).

The molecular weight of the recombinant collagen peptide is not particularly limited, but is preferably 2000 or more and 100,000 or less (2 kDa (kilodaltons) or more and 100 kDa or less), more preferably 2500 or more and 95000 or less (2.5 kDa or more and 95 kDa or less), still more preferably 5000 or more and 90,000 or less (5 kDa or more and 90 kDa or less), and most preferably 10,000 or more and 90,000 or less (10 kDa or more and 90 kDa or less).

The recombinant collagen peptide preferably has a repetition of a sequence represented by Gly-X-Y characteristic of collagen. Herein, a plurality of Gly-X-Ys may be the same or different. In Gly-X-Y, Gly represents glycine, and X and Y represent any amino acid (preferably any amino acid other than glycine). The sequence represented by Gly-X-Y, which is characteristic of collagen, is a highly specific partial structure in the amino acid composition and sequence of gelatin and collagen as compared with other proteins. In this portion, glycine accounts for about 1/3 of the total, and in the amino acid sequence, glycine is repeated one out of three. The glycine is the simplest amino acid, has little restriction on the arrangement of molecular chains, and greatly contributes to the regeneration of the helix structure during gelation. The amino acid represented by X and Y contains a large amount of imino acid (proline, oxyproline), and preferably occupies 10% to 45% of the whole amino acid. Preferably, 80% or more, more preferably 95% or more, and most preferably 99% or more amino acids of the sequence of the recombinant collagen peptide are Gly-X-Y repeat structures.

In general gelatin, charged and uncharged polar amino acids exist in a ratio of 1 : 1. Herein, the polar amino acid specifically refers to cysteine, aspartic acid, glutamic acid, histidine, lysine, asparagine, glutamine, serine, threonine, tyrosine, and arginine, and among these, the polar uncharged amino acid refers to cysteine, asparagine, glutamine, serine, threonine, and tyrosine. In the recombinant collagen peptide used in the present disclosure, the proportion of polar amino acids is 10 to 40%, preferably 20 to 30%, of the total amino acids that constitute the peptide. The proportion of the uncharged amino acid in the polar amino acid is preferably 5% or more and less than 20%, and more preferably 5% or more and less than 10%. Further, it is preferable that any one amino acid, preferably two or more amino acids, of serine, threonine, asparagine, tyrosine, and cysteine are not included in the sequence.

In general, in polypeptides, minimal amino acid sequences that serve as cell adhesion signals are known (for example, "Pathophysiology" Vol. 9, No. 7 (1990), page 527, published by Nagai Publishing Co., Ltd.). The recombinant collagen peptide used in the present disclosure may have two or more of these cell adhesion signals in one molecule. As a specific sequence, sequences of an RGD sequence, an LDV sequence, a REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and an HAV sequence represented in one-letter amino acid notation are preferable in that there are many types of adhering cells (SEQ ID NO: 2 to 9). An RGD sequence, a YIGSR sequence, a PDSGR sequence, a LGTIPG sequence, an IKVAV sequence, and an HAV sequence are more preferable, and the RGD sequence is particularly preferable. Among the RGD sequences, an ERGD sequence is preferable.

As the arrangement of the RGD sequence in the recombinant collagen peptide used in the present disclosure, it is preferable that the number of amino acids between RGDs is not uniform between 0 to 100, preferably between 25 to 60.

The content of this minimum amino acid sequence is preferably 3 to 50 pieces, more preferably 4 to 30 pieces, and particularly preferably 5 to 20 pieces in one molecule of the protein. The content is most preferably 12 pieces.

In the recombinant collagen peptide used in the present disclosure, the proportion of the RGD motif relative to the total number of amino acids is preferably at least 0.4%. If the recombinant gelatin includes 350 or more amino acids, it is preferable that each stretch of 350 amino acids includes at least one RGD motif. The proportion of RGD motifs relative to the total number of amino acids is more preferably at least 0.6%, still more preferably at least 0.8%, even more preferably at least 1.0%, particularly preferably at least 1.2%, and most preferably at least 1.5%. The number of RGD motifs in the recombinant peptide is preferably at least 4, more preferably 6, still more preferably 8, and particularly preferably 12 or more and 16 or less per 250 amino acids. The proportion of 0.4% of RGD motifs correspond to at least one RGD sequence per 250 amino acids. The number of RGD motifs is an integer, and thus a gelatin consisting of 251 amino acids must include at least 2 pieces of RGD sequences to meet the 0.4% feature. Preferably, the recombinant gelatin of the present invention includes at least 2 pieces of RGD sequences per 250 amino acids, more preferably at least 3 pieces of RGD sequences per 250 amino acids, and still more preferably at least 4 pieces of RGD sequences per 250 amino acids. A further aspect of the recombinant gelatin of the invention includes at least 4 pieces of RGD motifs, preferably 6 pieces, more preferably 8 pieces, and still more preferably 12 pieces or more and 16 pieces or less of RGD motifs.

The recombinant collagen peptide may be partially hydrolyzed.

More preferably, the polypeptide used in the present disclosure is represented by the following formula 2.

$$\text{Gly-Ala-Pro-[(Gly-X-Y)}_{63}\text{]}_3\text{-Gly}$$

In the formula, 63 pieces of X's each independently represent any of amino acids, and 63 pieces of Y's each independently represent any of amino acids. Herein, 63 pieces of Gly-X-Ys may be the same or different.

It is preferable that a plurality of naturally occurring sequence units of collagen is bound to form repeating units. The naturally occurring collagen referred to herein may be any naturally occurring collagen, but is preferably type I, type II, type III, type IV, or type V collagen. More preferably, it is type I, type II, or type III collagen. According to another form, the origin of the collagen is preferably human, bovine, porcine, mouse, or rat, and more preferably human.

The isoelectric point of the recombinant collagen peptide used in the present disclosure is preferably 5 to 10, more preferably 6 to 10, and still more preferably 7 to 9.5. The isoelectric point of the recombinant gelatin can be measured by measuring the pH after passing a 1% by mass peptide solution through a mixed crystal column of a cation and an anion exchange resin as described in isoelectric focusing (refer to Maxey, C.R. (1976; Phitogr. Gelatin 2, Editor Cox, P.J. Academic, London, Engl.).

Preferably, the recombinant collagen peptide is not deaminated.

Preferably, the recombinant collagen peptide does not have a telopeptide.

Preferably, the recombinant collagen peptide is a substantially pure polypeptide prepared with a nucleic acid encoding an amino acid sequence.

The recombinant collagen peptide particularly preferably has: (1) an amino acid sequence described in SEQ ID NO: 1; or (2) an amino acid sequence having 80% or more (preferably 90% or more, more preferably 95% or more, and particularly preferably 98% or more) sequence identity to the amino acid sequence described in SEQ ID NO: 1 and having bioaffinity.

In the present description, the sequence identity refers to a value calculated by the following formula.

$$\text{Sequence identity \% = [(number of identical residues)/(alignment length)]} \times 100$$

The sequence identity between two amino acid sequences can be determined by any method known to those skilled in the art, and can be determined using the BLAST (Basic Local Alignment SearchTool) program (J. Mol. Biol. 215: 403-410, 1990) or the like.

The recombinant collagen peptide may consist of an amino acid sequence in which one or several amino acids are deleted, substituted, or added in the amino acid sequence described in SEQ ID NO: 1, and have an amino acid sequence having bioaffinity.

"One or several" in "amino acid sequence in which one or several amino acids are deleted, substituted, or added" means preferably 1 to 20, more preferably 1 to 10, still more preferably 1 to 5, and particularly preferably 1 to 3.

The recombinant collagen peptide can be produced by a genetic recombination technique known to those skilled in the art, and can be produced, for example, according to the methods described in EP 1014176 A2, US 6992172, WO 2004/85473, WO 2008/103041, and the like. Specifically, a gene encoding the amino acid sequence of a predetermined recombinant collagen peptide is obtained, and incorporated into an expression vector to produce a recombinant expression vector, which is then introduced into an appropriate host to produce a transformant. The obtained transformant is cultured in an appropriate medium to produce the recombinant collagen peptide, and thus the recombinant collagen peptide produced from the culture is collected to allow preparation of the recombinant collagen-like peptide used in the present invention.

In some embodiments, the gelling agent may be included in a storage solution at a concentration of about 1% (w/w) or more, such as about 1% (w/w) to about 10% (w/w), about 2% (w/w) to about 10% (w/w), about 1% (w/w) to about 9% (w/w), about 2% (w/w) to about 9% (w/w), about 1% (w/w) to about 8% (w/w), about 2% (w/w) to about 8% (w/w), about 1% (w/w) to about 7% (w/w), about 2% (w/w) to about 7% (w/w), about 1% (w/w) to about 6% (w/w), about 2% (w/w) to about 6% (w/w), about 1% (w/w) to about 5% (w/w), about 2% (w/w) to about 5% (w/w), about 1% (w/w) to about 4% (w/w), about 2% (w/w) to about 4% (w/w), about 1% (w/w) to about 3% (w/w), or about 2% (w/w) to about 3% (w/w). In some embodiments, the gelling agent may be included in a storage solution at a concentration of about 2.5% (w/w). In a further embodiment, the gelling agent may be included in a storage solution at concentrations of $2.5 \pm 1.5\%$ (w/w), $2.5 \pm 1\%$ (w/w), or $2.5 \pm 0.5\%$ (w/w).

In some embodiments, the storage solution may be a cell culture solution, a saline solution, or an electrolyte solution. In some embodiments, the pH of the storage solution may be about 5.0 to about 8.0, about 6.0 to about 8.0, about 6.0 to about 7.5, about 6.1 to about 7.4, about 6.2 to about 7.4, about 6.3 to about 7.4, about 6.4 to about 7.4, about 6.4 to about 7.3, about 6.4 to about 7.2, about 6.4 to about 7.1, about 6.4 to about 7.0, about 6.4 to about 6.9, about 6.4 to about 6.8, about 6.4 to about 6.7, about 6.4 to about 6.6, or about 6.4 to about 6.5. In a certain embodiment, the pH of the storage solution may be about 6.4 to about 7.4.

In some embodiments, the storage solution may be a cell culture solution. The storage solution may or may not include growth factors. In the method of the present disclosure, cell growth does not occur with or without growth factors because the cells are stored at low temperatures. Examples of the storage solution include MEMα, physiological saline, PBS, DMEM, CELSIOR (registered trademark) Cold Storage Solution (Celsior solution) (Waters Medical Systems LLC), organ tissue storage solution ETK (registered trademark) (ETK solution) (Otsuka Pharmaceutical Co., Ltd.), and Belzer UW (registered trademark) cold storage solution (UW solution) (Asteral Pharma Inc.), but are not limited thereto.

In some embodiments, the gel-state maintaining temperature may be about 0°C to about 37°C, about 0°C to about 30°C, about 0°C to about 24°C, about 0°C to about 20°C, about 0°C to about 15°C, or about 0°C to about 10°C. In a further embodiment, the gel-state maintaining temperature may be about 4°C. In a certain embodiment, the gel-state maintaining temperature may be $4 \pm 2.0$°C, $4 \pm 1.5$°C, $4 \pm 1.0$°C, or $4 \pm 0.5$°C.

In some embodiments, the gelling temperature and the gel-state maintaining temperature may be the same or different.

In some embodiments, the cells to be stored may be mesenchymal stem cells of any origin (for example, from bone marrow, adipose tissue, placental tissue, synovial tissue, umbilical cord tissue (for example, cord blood), dental pulp, or amniotic membrane) or mesenchymal stem cells differentiated from ES cells or iPS cells. In a certain embodiment, the mesenchymal stem cell may be a mesenchymal stem cell derived from bone marrow. In a further embodiment, the stem cell may be a cell used in cell infusion therapy and drug discovery research. In a further embodiment, the cell may be obtained from a subject undergoing cell infusion therapy.

The storage method of the present disclosure may further include a step of subjecting the storage solution to a non-gelling treatment. The step of non-gelling treatment includes increasing the temperature of the storage solution to a melting point or more of the gelling agent. The melting point of the gelling agent may vary depending on the concentration, pH, and the like of the gelling agent, but the melting point of the gelling agent may be 37°C or less. The temperature at the time of the non-gelling treatment is equal to the melting point of the gelling agent or more than the melting point, and can be appropriately determined. In a certain embodiment, the gelling agent is a recombinant collagen peptide, and the temperature of the storage solution in the step of non-gelling treatment may be about 10°C to about 30°C, about 15°C to 25°C, or about 20°C.

The storage method of the present disclosure may further include a step of collecting stem cells. In some embodiments, the step of collecting stem cells may include diluting the storage solution after storage 2- to 20-fold, such as about 2-fold, about 3-fold, about 4-fold, about 5-fold, about 6-fold, about 7-fold, about 8-fold, about 9-fold, about 10-fold, about 11-fold, about 12-fold, about 13-fold, about 14-fold, about 15-fold, about 16-fold, about 17-fold, about 18-fold, about 19-fold, or about 20-fold. In a preferable embodiment, the storage solution after storage may be diluted about 5-fold or more up to about 20-fold. In some embodiments, the step of collecting stem cells may include separating (for example, centrifugation) the stem cells and the storage solution. Without wishing to be bound by theory, if the temperature of the storage solution is raised to the melting point or more to bring the storage solution into a non-gelled state, the viscosity of the storage solution is high and the collecting rate of cells is low, and thus it is preferable to dilute the storage solution, and typically, diluting 5-fold or more causes the collecting rate of cells to be high. The solution for dilution may be a storage solution without a gelling agent.

In some embodiments, the stem cells may be stored at a cell density of about $1 \times 10^5$ to about $1 \times 10^8$ cells/ml, preferably about $5 \times 10^5$ to about $5 \times 10^6$ cells/ml, and more preferably about $1 \times 10^6$ to about $1 \times 10^7$ cells/ml.

(Storing agent for stem cells)

In another aspect, the present disclosure provides a composition for storing stem cells (for example, a storage solution) including a gelling agent. The composition of the present disclosure (for example, a storage solution) may be used in storing stem cells at a pH of about 5.0 to about 8.0. The compositions of the present disclosure (for example, a storage solution) may be used so as to store stem cells at a gel-state maintaining temperature of the gelling agent. The compositions of the present disclosure may have one or more features in the above embodiments.

(Stem-cell storing kit)

In a further aspect, the present disclosure provides a kit for storing stem cells, the kit including: (1) a storage solution including a gelling agent; and (2) a buffer solution for adjusting a pH of the storage solution to about 5.0 to about 8.0, wherein the stem cells are stored at a gel-state maintaining temperature. The kit of the present disclosure may have one or more features in the above embodiments.

(Cell formulation)

In a further aspect, the present disclosure may provide a formulation including a gelling agent and stem cells, wherein the pH is about 5.0 to about 8.0. The formulation may be used so as to store stem cells at a gel-state maintaining temperature of the gelling agent. The formulation may be gelled. The formulation of the present disclosure may be a Ready-to-Use formulation.

(Storage of tissue or organ)

In a further aspect, the present disclosure provides a method for storing a tissue or organ, the method including: 1) a step of providing a storage solution including a gelling agent at a temperature more than a gelling temperature in a non-gelled state and having a pH of about 5.0 to about 8.0; 2) a step of putting the a tissue or organ into the storage solution and then lowering a temperature to the gelling temperature of the gelling agent; and 3) a step of storing the storage solution including the tissue or organ at a gel-state maintaining temperature of the gelling agent.

In further another aspect, the present disclosure provides a kit for storing a tissue or organ, the kit including: (1) a storage solution including a gelling agent; and (2) a buffer solution for adjusting a pH of the storage solution to about 5.0 to about 8.0, wherein the tissue or organ are stored at a gel-state maintaining temperature. A tissue and an organ is aggregates of cells, and as described in the present description, the storage of cells is achieved by the technology of the present disclosure, and thus a tissue and an organ that is the aggregates can be stored by applying the technology of the present disclosure under similar conditions. In doing so, it is understood that those skilled in the art can appropriately adjust the optimum conditions in light of the disclosure of the present description according to the feature of each of various tissues and/or organs.

In further another aspect, the present disclosure provides a composition for storing a tissue or organ, the composition including a gelling agent. In the storage of a tissue or organ, a storage solution for a tissue or organ can be used as the storage solution. Examples of the storage solution for a tissue or organ include, but are not limited to, an organ tissue storage solution ETK (registered trademark) (ETK solution) (Otsuka Pharmaceutical Co., Ltd.), a Belzer UW (registered trademark) cold storage solution (UW solution) (Asteral Pharma Inc.), and a Celsior solution. A gelling agent may be dissolved in the storage solution and used.

In some embodiments, the ETK solution may include sodium gluconate, potassium dihydrogen phosphate, dipotassium phosphate, hydroxyethyl starch, trehalose hydrate, and potassium hydroxide. In a certain embodiment, the ETK solution may include 21.814 g of sodium gluconate, 0.885 g of potassium dihydrogen phosphate, 3.222 g of dipotassium phosphate, 30.0 g of hydroxyethyl starch, 45.3 g of trehalose hydrate, and an appropriate amount of potassium hydroxide in 1000 mL.

In some embodiments, the UW solution may include pentafluorose, lactobionic acid (as lactone), potassium dihydrogen phosphate, magnesium sulfate heptahydrate, raffinose pentahydrate, adenosine, allopurinol, total glutathione, potassium hydroxide, sodium hydroxide/hydrochloric acid (for pH adjustment), and water for injection. In a certain embodiment, the UW solution may include 50 g/L pentafraction, 35.83 g/L lactobionic acid (as lactone), 3.4 g/L potassium dihydrogen phosphate, 1.23 g/L magnesium sulfate heptahydrate, 17.83 g/L raffinose pentahydrate, 1.34 g/L adenosine, 0.136 g/L allopurinol, 0.922 g/L total glutathione, 5.61 g/L potassium hydroxide, sodium hydroxide/hydrochloric acid (adjusted to pH 7.4), and an appropriate amount of water for injection.

In some embodiments, Celsior solution may include mannitol, lactobionic acid, glutamic acid, histidine, calcium chloride, potassium chloride, magnesium chloride, sodium hydroxide, reduced glutathione, and water for injection. In a certain embodiment, Celsior solution may include 10.930 g/L mannitol, 28.664 g/L lactobionic acid, 2.942 g/L glutamic acid, 4.650 g/L histidine, 0.037 g/L calcium chloride, 1.118 g/L potassium chloride, 2.642 g/L magnesium chloride, 4.000 g/L sodium hydroxide, 0.921 g/L reduced glutathione, and an appropriate amount of water for injection.

One or more embodiments described with respect to the storage of the cells are employed as embodiments in aspects of tissue or organ storage, where appropriate.

(Conveying method)

In a further aspect, the present disclosure is a method for conveying stem cells and provides the method including: 1) a step of providing a storage solution including a gelling agent at a temperature more than a gelling temperature in a non-gelled state and having a pH of about 5.0 to about 8.0; 2) a step of putting the stem cells into the storage solution and then lowering a temperature to the gelling temperature of the gelling agent; and 3) a step of conveying the storage solution including the stem cells at a gel-state maintaining temperature of the gelling agent. The method of the present disclosure can perform conveying that can withstand conveying conditions such as vibration while maintaining cell viability in an unfrozen state. Conveying may include transportation by land, sea, air, or a combination thereof.

(Cells)

In a further aspect, the present disclosure provides a composition for treating or preventing a disease, disorder, or condition in a subject, the composition including stem cells stored according to the methods above, or stem cells conveyed according to the methods above. In some embodiments, those that may be treated for regenerative medicine using stem cells include, but are not limited to, severe burns, spinal cord injury, head injury, cerebral infarction, cerebral hemorrhage, and Parkinson's disease.

(Syringe formulation)

In a further aspect, the present disclosure provides a syringe formulation including a suspension of stem cells and a syringe, wherein the syringe formulation can be administered to a subject after storage or conveying of the stem cells by the method without further processing.

(Treatment method)

In a further aspect, the present disclosure provides a method for treating or preventing a disease, disorder, or condition in a subject, the method including: a step of storing or conveying stem cells according to the above method; and a step of administering the stored or conveyed stem cells to the subject in need thereof.

(Use)

In a further aspect, the present disclosure provides the use of stem cells stored or conveyed according to the above methods in the production of pharmaceuticals (for example, the syringe formulation) for treating or preventing a disease, disorder, or condition in a subject.

The present disclosure has been described above with reference to preferable embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described in the present description, but is limited only by the claims.

Examples

Hereinafter, the present disclosure will be described more specifically based on examples.

(Measurement method)

In the following examples, the measurement of the number of cells was performed twice after storage and after reseeding, and the cell viability after storage was calculated from "cell viability after storage = number of cells after storage/number of cells before storage", and the cell viability after reseeding was calculated from "cell viability after reseeding = number of cells after reseeding/number of cells before storage" by seeding the previous cells in a flask after storage, adding a culture solution (MEMα + PL (platelet lysate)), and culturing for 72 hours.

For the measurement of the number of cells, living cells and dead cells were fluorescently measured using a Luna Automated Cell counter (https://logosbio.com/automated-cell-counters/brightfield/luna).

(Example 1: Possible survival equivalent to or more than cryostorage despite of vibration at 4°C for 72 hours in the presence of RCP protein)

(Materials and methods)

· Materials

The following commercially available reagents or cells were used.

RCP (recombinant collagen peptide): Reagent manufactured by Fujifilm Corporation (https://www.fujifilm.com/jp/ja/business/materials/rm/rcp)

Human bone marrow stem cell PL (platelet lysate): Collected from health volunteer at Hokkaido University, approved by Ethics Committee, and obtained consent form

> **MEMα:** Catalog No.: Invitrogen, 32571-036, USA
> **MEMαPL solution:** PL is self-produced from platelet liquid collected from a health volunteer
> Cryostorage solution Bambanker (registered trademark) Catalog No.: CS-02-001 (Nippon Genetics Co., Ltd.)

· Methods

500,000 human bone marrow stem cells separated and cultured from a bone marrow liquid obtained from a health volunteer were each (1) **diluted into MEMα to** adjust the pH to around 7.0, (2) RCP (final concentration 2.5%) was dissolved in **MEMαPL liquid to adjust the pH to around 7.0, and (3)** diluted into a cryostorage solution Bambanker (registered trademark) (Nippon Genetics Co., Ltd.). Thereafter, (1) and (2) were stored in a 4°C refrigerator with vortex machine (Heathrow Scientific, HS120318, 3000 rpm) for 72 hours while applying vibration thereto. (3) was left to stand for storage in a freezer for experiment at -80°C.

(Results)

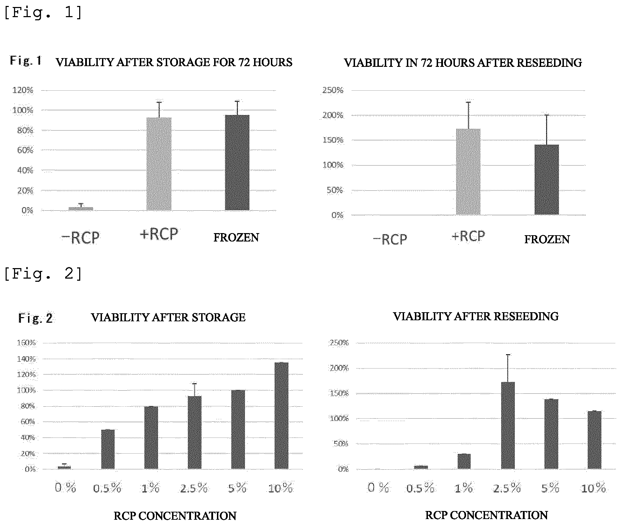

The results are shown in FIG. 1. In the absence of RCP, the viability of cells to which vibration was applied at 4°C was almost 0%, and growth was not observed even after re-seeding. On the other hand, the cells in the storage solution gelled by RCP were able to growth to nearly 1.6 times even after reseeding at a viability of 95%, which was equivalent to the result of cryostorage.

(Example 2: Investigation of RCP concentration in storage solution)

(Materials and methods)

500,000 human bone marrow stem cells obtained by separating and culturing a bone marrow liquid obtained from a health volunteer were dissolved in an RCP solution having a concentration of 0, 0.5, 1.0, 2.5, 5.0, or 10.0% to adjust the pH to around 7.0, and this solution was stored in a 4°C refrigerator for 72 hours while being vibrated with vortex machine (Heathrow Scientific, HS120318, 3000 rpm). After 72 hours, the mixture was liquefied at 20°C, and the number of cells was measured. Liquefying was performed as follows.

The gel was left to stand in an incubator at 20°C to return to a liquid. Thereafter, the liquid gel was diluted by adding a 5-**fold amount of medium (MEMα), and** centrifuged to pellet the cells, which were collected at a bottom of a centrifuge tube. All the supernatant was aspirated to remove RCP as a component of the gel, a small amount **(1 ml) of a medium (MEMα) was added, and the number of cells was counted under** stirring. When the mixture was left to stand at 20°C to be liquefied and centrifuged as it was, and the cells were dropped down as pellets, some of the cells remained in the supernatant, and the collecting rate was deteriorated.

(Results)

The results are shown in FIG. 2. Although the viability after storage increased depending on the concentration of RCP, the viability after reseeding was the highest at 2.5%.

(Example 3: Investigation of medium dissolving RCP)

(Materials and methods)

In order to store 500,000 human bone marrow stem cells obtained by separating and culturing a bone marrow liquid obtained from a health volunteer, a medium for **diluting 2.5% of RCP was dissolved in MEMαPL, physiological saline, Belzer UW cold** storage solution (organ storage solution, Astellas Pharma Inc.), Hypothermosol-FRS (low temperature cell storage reagent, Sigma-Aldrich Co. LLC), phosphorylated saline (PBS, commercially available product), or a 5% glucose solution (commercially available product) to adjust the pH to around 7.0, and then stored in a 4°C refrigerator with vortex machine for 72 hours while applying vibration. After 72 hours, the mixture was liquefied at 20°C, and the number of cells was measured.

(Results)

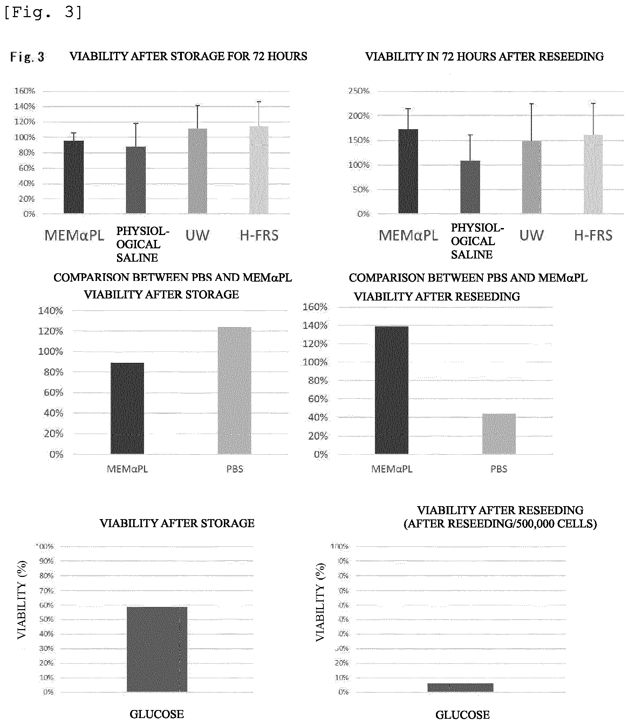

The results are shown in FIG. 3. It is considered that any solvent did not adversely affect the viability after storage. PBS and glucose had low viability after seeding. Therefore, depending on the type of the storage solution, it is desirable not to perform reseeding after storage. In any case, a high viability was maintained even when any of the storage solutions was used, and thus reseeding may not be performed as long as the desired number of cells is achieved.

(Example 4: Investigation of temperature during storage)

(Materials and methods)

500,000 human bone marrow stem cells obtained by separating and culturing a bone marrow liquid obtained from **a health volunteer were stored in MEMαPL (pH 7.0)** with 2.5% RCP dissolved at -20°C, 4°C, 24°C, or 37°C for 72 hours. After 72 hours, the mixture was liquefied at 20°C, and the number of cells was measured. Thereafter, all the cells were seeded in a flask **and cultured in a culture solution (MEMaPL) for 72** hours to confirm the growth capability.

(Results)

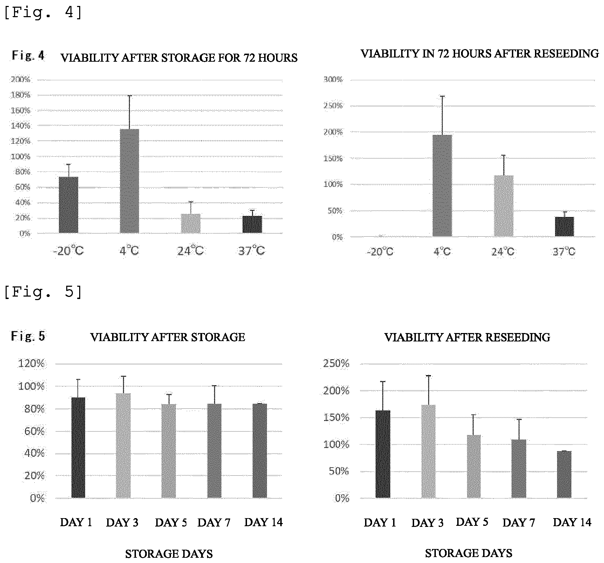

The results are shown in FIG. 4. At 4°C, the cell viability was maintained at 100% or more after storage for 72 hours and in 72 hours after reseeding. At -20°C, the cell viability after storage for 72 hours was about 70%, but in 72 hours after reseeding, almost no cells survived. This suggests that the cells were alive immediately after storage, but had some damage. In the storage method of the present disclosure, it is suggested that storage in an unfrozen state is preferable. In addition, in the case of storage at 24°C, the cell viability after storage for 72 hours was as low as about 20%, but the cell viability after reseeding was about 100%. At 37°C, the cell viability after storage for 72 hours and in 72 hours after reseeding was 50% or less. As described above, the temperature at the time of storage was a temperature at which freezing did not occur (for example, 0°C or more), preferably less than 37°C (for example, 24°C or less), and 4°C was optimal.

(Example 5: Investigation of storage days)

(Materials and methods)

500,000 human bone marrow stem cells obtained by separating and culturing a bone marrow liquid obtained from **a health volunteer were stored in MEMαPL (pH 7.0)** with 2.5% RCP dissolved for 1 day, 3 days, 5 days, 7 days, or 14 days at 4°C while being vibrated. After a predetermined number of days, the mixture was liquefied at 20°C, and the number of cells was measured.

(Results)

The results are shown in FIG. 5. Although the viability after storage was substantially the same for all days, the viability after reseeding was particularly good for 1 to 3 days, and thereafter, the viability decreased with time, but the viability of about 80% was maintained even for 14 days.

(Example 6: Investigation of type of gelling agent)

(Materials and methods)

In order to store 500,000 human bone marrow stem cells obtained by separating and culturing a bone marrow liquid obtained from a health volunteer, 2.5, 5%, and 10% of beMatrix gelatin (Nitta Gelatin Inc., LS-250), cook gelatin (Morinaga & Co., Ltd.), **jelly gelatin powder (Maruha Nichiro Corporation), and RCP were dissolved in MEMaPL,** and the pH was adjusted to about 7.0. The beMatrix and the RCP were stored at 4°C while being vibrated, and the cook gelatin and the jelly gelatin powder were left to stand for storage, and then the number of cells was measured.

(Results)

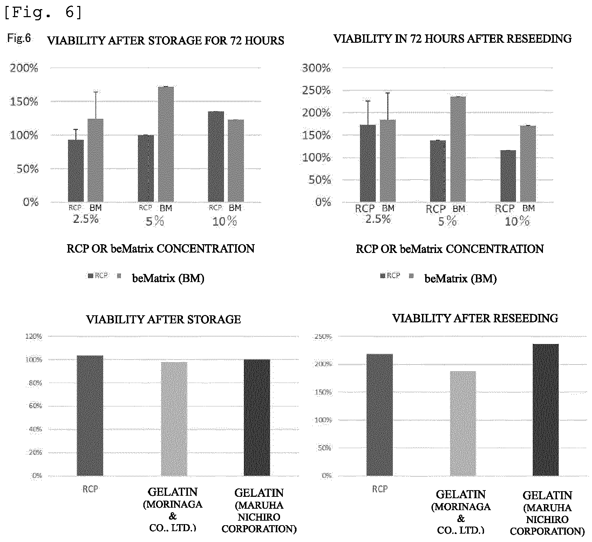

The results are shown in FIG. 6. Cell storage was possible with all the gelatins: beMatrix gelatin, cook gelatin, and jelly. Therefore, it is considered to be important that the storage solution is gelled.

(Example 7: Investigation of pH during storage)

(Materials and methods)

In order to store 500,000 human bone marrow stem cells obtained by separating and culturing a bone marrow liquid obtained from a health volunteer, a storage solution obtained by dissolving 2.5% RCP, cook gelatin, and rice gelatin powder **in MEMαPL was** used. The pH was adjusted to 4.4 to 8.4, and the cells were vibrated or left to stand for storage at 4°C, and then the number of cells was measured.

(Results)

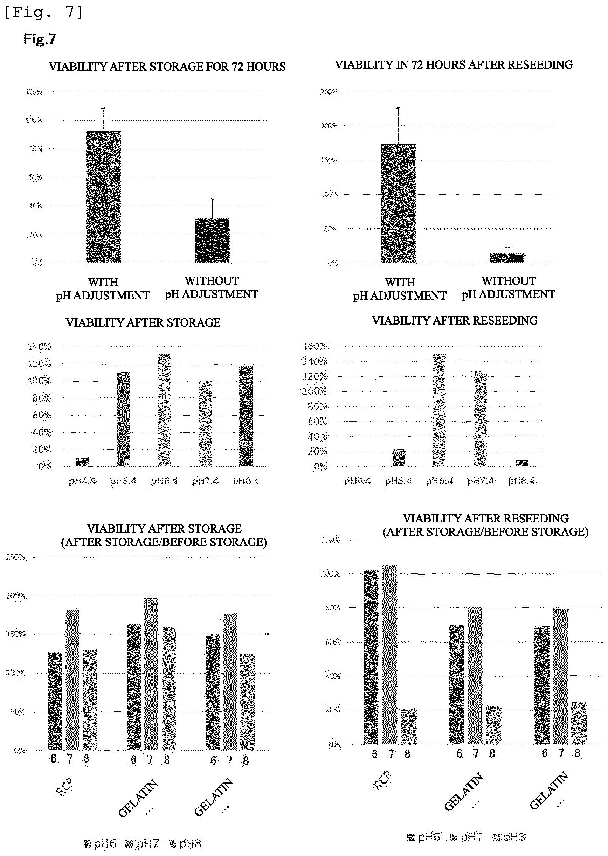

The results are shown in FIG. 7. In the absence of pH adjustment (about 9), the cells hardly survived. In addition, a high viability was shown at pH 5.4 to 8.4, but the cells could hardly survive at pH 4.4. After seeding, viability was high at pH 6.4 and 7.4.

(Example 8: Storage of a tissue and an organ)

(Materials and methods)

· Materials

Tissue organ storage solution ETK (registered trademark) (ETK solution): Otsuka Pharmaceutical Co., Ltd., or Belzer UW (registered trademark) cold storage solution (UW Solution): Asteral Pharm Inc.
RCP (recombinant collagen peptide): Reagent manufactured by Fujifilm Corporation (https://www.fujifilm.com/jp/ja/business/materials/rm/rcp)

· Methods

RCP (final concentration 2 to 10%) was dissolved in ETK solution or UW solution to adjust the pH to around 7. A tissue (for example, corneal tissue, skin tissue, and the like) or an organ (for example, lung, kidney, liver, pancreas, and the like) was placed in a container containing the ETK solution or the UW solution and aseptically sealed, and the container containing the tissue or organ was cooled.

(Example 9: Cell conveying)

In the present example, conveying by an airplane was actually performed, and the viability after transportation was measured.

(Materials and methods)

· Materials

**MEM$\alpha$ + 2.5%RCP**
50 ml Falcon tube or 100 ml sterile bottle
Bone marrow stem cells
Standard constant temperature transportation package TACPack0208F (Tamai Kasei Co., Ltd.)

· Methods

**RCP 2.5% (final concentration) was dissolved in MEM$\alpha$ and adjusted to a pH** of 6.05 to prepare a storage solution. The cells were placed in a container (50 ml falcon tube) containing the storage solution so that the cell density was set to $1.0 \times 10^6$ cells/ml, and the inside was replaced with air of 5% $CO_2$ ($O_2$ 20% $N_2$ 75%), then the container was aseptically sealed, and cooled to 4°C. The container was horizontally placed in a standard constant temperature transportation package TACPack0208F (Tamai Kasei Co., Ltd.), and transportation was performed while maintaining the temperature at 4°C. The cells were shipped from Sapporo, arrived at Wakayama two days later, and stored at 4°C per day, and then the cells were shipped from Wakayama three days later and arrived in Sapporo five days later. The transportation involved land transportation and air transportation. As a control, cells were left to stand for storage.

(Results)

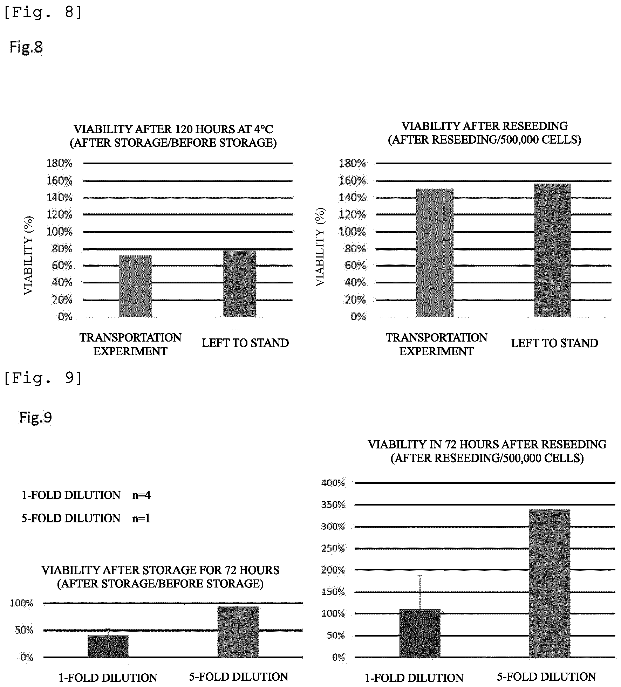

The results are shown in FIG. 8. As shown, the transported cells exhibited cell viability comparable to that of the cells that were left to stand for storage. In addition, it was confirmed that the stored cells were normally differentiated into fat, bone, and cartilage. Therefore, the storage solution of the present invention allows transportation that can withstand transportation conditions such as vibration while maintaining cell viability in an unfrozen state.

(Example 10: Change in viability by dilution of storage solution at the time of cell collection)

In the present example, it was confirmed whether the cell viability was changed by diluting the storage solution when the cells were collected.

(Materials and methods)

· Materials

**MEM$\alpha$ + 2.5%RCP**
50 ml Falcon tube or frozen bag (registered trademark) (Nipro Corporation)
Human bone marrow stem cells

· Methods

**RCP 2.5% (final concentration) was dissolved in MEM$\alpha$ and adjusted to a pH** of 7.4 to 8.0 to prepare a storage solution. The cells were placed in a container (50 ml falcon tube or frozen bag (registered trademark)) containing the storage solution so that the cell density was set to $1.0 \times 10^6$ cells/ml, and the container was aseptically sealed, cooled to 4°C, and left to stand for storage at 4°C for 72 hours. Melting was performed at 37°C for 10 minutes to 3 hours.
After storage, diluting was performed (1- or **5-fold dilution) by adding MEM$\alpha$** and centrifugation was performed to pellet the cells, which were collected under centrifuge tubes. All the supernatant was aspirated to remove RCP as a component of **the gel, a small amount (1 ml) of a medium (MEM$\alpha$) was added, and the number of cells** was counted under stirring.

(Results)

The results are shown in FIG. 9. As shown, diluting the storage solution 5-fold in collecting the cells increased cell viability compared to 1-fold dilution. Without wishing to be bound by theory, it is considered that the increase in cell viability by 5-fold dilution is due to the viscosity of the gelling agent. Even if centrifugation is performed, it is assumed that cells cannot be sufficiently precipitated with a liquid in a state of having high viscosity, and the collecting rate is lowered. It is considered that diluting 5-fold or more lowers the viscosity, and thus almost all cells can be precipitated and collected using a commonly used centrifugation method (1000 G).

Although the present disclosure has been illustrated using preferable embodiments of the present disclosure as described above, it is understood that the scope of the present disclosure should be interpreted only by the claims. It is understood that the patents, patent applications, and documents cited in the present description are to be incorporated by reference in the present description in the same manner as their content is specifically described in the present description.

This application claims the benefit of priority of Japanese Patent Application No. 2021-126706 filed on August 2, 2021, which is incorporated in the present description by reference in its entirety.

Industrial Applicability

Provided is a storage method capable of maintaining cells at a high viability. The stored cells are used for cell infusion therapy, and thus are available in the field of pharmacy and the like.

Sequence Listing Free Text

SEQ ID NO: 1: Amino acid sequence of recombinant collagen peptide (RCP)
SEQ ID NO: 2: Cell adhesion signal sequence (REDV)
SEQ ID NO: 3: Cell adhesion signal sequence (YIGSR)
SEQ ID NO: 4: Cell adhesion signal sequence (PDSGR)
SEQ ID NO: 5: Cell adhesion signal sequence (RYVVLPR)
SEQ ID NO: 6: Cell adhesion signal sequence (LGTIPG)
SEQ ID NO: 7: Cell adhesion signal sequence (RNIAEIIKDI)
SEQ ID NO: 8: Cell adhesion signal sequence (IKVAV)
SEQ ID NO: 9: Cell adhesion signal sequence (DGEA)

**Claims**

1.   A method for storing stem cells, the method comprising:

1) a step of providing a storage solution including a gelling agent at a temperature more than a gelling temperature and having a pH of about 5.0 to about 8.0;
2) a step of putting the stem cells into the storage solution and then lowering a temperature to the gelling temperature of the gelling agent; and
3) a step of storing the storage solution including the stem cells at a gel-state maintaining temperature of the gelling agent.

2.   The method according to claim 1, wherein the gelling agent includes a peptide.

3.   The method according to claim 1, wherein the gelling agent includes collagen, denatured collagen, collagen-like peptide, gelatin, or any combination thereof.

4.   The method according to claim 1, wherein the gelling agent includes a collagen-like peptide, the collagen-like peptide includes a repeat of a sequence represented by Gly-X-Y, and X and Y are each independently identical or different amino acids.

5.   The method according to any one of claims 1 to 4, wherein the gelling agent is included in the storage solution at a concentration of about 1% (w/w) or more.

6.   The method according to claim 5, wherein the gelling agent is included in the storage solution at a concentration of about 1% (w/w) to about 10% (w/w).

7. The method according to claim 6, wherein the gelling agent is included in the storage solution at a concentration of about 2.5% (w/w).

8. The method according to any one of claims 1 to 7, wherein the storage solution is a cell culture solution, physiological saline, or an electrolyte solution.

9. The method according to any one of claims 1 to 8, wherein the pH of the storage solution is about 6.0 to about 7.5.

10. The method according to claim 9, wherein the pH of the storage solution is about 6.4 to about 7.4.

11. The method according to any one of claims 1 to 10, wherein the gel-state maintaining temperature is about 0°C to about 37°C.

12. The method according to claim 11, wherein the gel-state maintaining temperature is about 0°C to about 24°C.

13. The method according to any one of claims 1 to 12, wherein the stem cells are mesenchymal stem cells.

14. The method according to claim 13, wherein the mesenchymal stem cells are mesenchymal stem cell stem cells derived from bone marrow, adipose tissue, placental tissue, synovial tissue, umbilical cord tissue (for example, cord blood), dental pulp, or amniotic membrane, or mesenchymal stem cells differentiated from ES cells or iPS cells.

15. The method according to claim 14, wherein the mesenchymal stem cells are mesenchymal stem cells derived from bone marrow.

16. The method according to any one of claims 1 to 15, wherein the stem cells are cells used in cell infusion therapy and drug discovery research and are obtained from a subject receiving the cell infusion therapy.

17. The method according to any one of claims 1 to 15, further comprising a step of non-gelling treatment.

18. The method according to claim 17, wherein the step of non-gelling treatment includes increasing a temperature to a melting point of the gelling agent or a temperature higher than the melting point.

19. The method according to any one of claims 1 to 18, further comprising a step of collecting the stem cells.

20. The method according to claim 19, wherein the step of collecting the stem cells includes diluting the storage solution 2-fold to 20-fold and separating the stem cells and the storage solution.

21. The method according to any one of claims 1 to 20, wherein the stem cells are stored at a cell density of about $1 \times 10^6$ to about $1 \times 10^7$ cells/ml.

22. A kit for storing stem cells, the kit comprising:

    (1) a storage solution including a gelling agent; and
    (2) a buffer solution for adjusting a pH of the storage solution to about 5.0 to about 8.0,

    wherein the stem cells are stored at a gel-state maintaining temperature.

23. A composition for storing stem cells, the composition comprising a gelling agent.

24. The composition according to claim 23, wherein the composition is used at a pH of about 5.0 to about 8.0 in storing stem cells.

25. The composition according to claim 23 or 24, wherein the composition is used to store stem cells at a gelling temperature of the gelling agent.

26. A formulation comprising a gelling agent and stem cells, wherein a pH is about 5.0 to about 8.0.

27. The formulation according to claim 26, wherein the formulation is a Ready-to-Use formulation.

**28.** A method for storing a tissue or organ, the method comprising:

1) a step of providing a storage solution including a gelling agent at a temperature more than a gelling temperature and having a pH of about 5.0 to about 8.0;
2) a step of putting the tissue or organ into the storage solution and then lowering a temperature to the gelling temperature of the gelling agent; and
3) a step of storing the storage solution including the tissue or organ at a gel-state maintaining temperature of the gelling agent.

**29.** A kit for storing a tissue or organ, the kit comprising:

(1) a storage solution including a gelling agent; and
(2) a buffer solution for adjusting a pH of the storage solution to about 5.0 to about 8.0,

wherein the tissue or organ are stored at a gel-state maintaining temperature.

**30.** A composition for storing a tissue or organ, the composition comprising a gelling agent.

**31.** The composition according to claim 30, wherein the composition is used at a pH of about 5.0 to about 8.0 in storing a tissue or organ.

**32.** A method for conveying stem cells, the method comprising:

1) a step of providing a storage solution including a gelling agent at a temperature more than a gelling temperature and having a pH of about 5.0 to about 8.0;
2) a step of putting the stem cells into the storage solution and then lowering a temperature to the gelling temperature of the gelling agent; and
3) a step of conveying the storage solution including the stem cells at a gel-state maintaining temperature of the gelling agent.

**33.** A gelling agent for storing stem cells, wherein the gelling agent is used at a gel-state maintaining temperature of the gelling agent at a pH of about 5.0 to about 8.0 in storing the stem cells.

**34.** A gelling agent for storing a tissue or organ, wherein the gelling agent is used at a gel-state maintaining temperature of the gelling agent at a pH of about 5.0 to about 8.0 in storing the tissue or organ.

[Fig. 1]

Fig. 1  VIABILITY AFTER STORAGE FOR 72 HOURS

VIABILITY IN 72 HOURS AFTER RESEEDING

[Fig. 2]

Fig. 2  VIABILITY AFTER STORAGE

VIABILITY AFTER RESEEDING

RCP CONCENTRATION

RCP CONCENTRATION

[Fig. 3]

Fig. 3    VIABILITY AFTER STORAGE FOR 72 HOURS            VIABILITY IN 72 HOURS AFTER RESEEDING

COMPARISON BETWEEN PBS AND MEMαPL          COMPARISON BETWEEN PBS AND MEMαPL
VIABILITY AFTER STORAGE                            VIABILITY AFTER RESEEDING

VIABILITY AFTER STORAGE

VIABILITY AFTER RESEEDING
(AFTER RESEEDING/500,000 CELLS)

[Fig. 4]

**Fig. 4** VIABILITY AFTER STORAGE FOR 72 HOURS

VIABILITY IN 72 HOURS AFTER RESEEDING

[Fig. 5]

**Fig. 5** VIABILITY AFTER STORAGE

VIABILITY AFTER RESEEDING

EP 4 382 598 A1

[Fig. 6]

Fig.6  VIABILITY AFTER STORAGE FOR 72 HOURS          VIABILITY IN 72 HOURS AFTER RESEEDING

RCP OR beMatrix CONCENTRATION

■RCP  ■ beMatrix (BM)

RCP OR beMatrix CONCENTRATION

■RCP  ■ beMatrix (BM)

VIABILITY AFTER STORAGE                    VIABILITY AFTER RESEEDING

[Fig. 7]

Fig.7

VIABILITY AFTER STORAGE FOR 72 HOURS

VIABILITY IN 72 HOURS AFTER RESEEDING

VIABILITY AFTER STORAGE

VIABILITY AFTER RESEEDING

VIABILITY AFTER STORAGE
(AFTER STORAGE/BEFORE STORAGE)

VIABILITY AFTER RESEEDING
(AFTER STORAGE/BEFORE STORAGE)

[Fig. 8]

Fig.8

VIABILITY AFTER 120 HOURS AT 4°C
(AFTER STORAGE/BEFORE STORAGE)

VIABILITY AFTER RESEEDING
(AFTER RESEEDING/500,000 CELLS)

[Fig. 9]

Fig.9

1-FOLD DILUTION   n=4

5-FOLD DILUTION   n=1

VIABILITY AFTER STORAGE FOR 72 HOURS
(AFTER STORAGE/BEFORE STORAGE)

VIABILITY IN 72 HOURS AFTER RESEEDING
(AFTER RESEEDING/500,000 CELLS)

# EP 4 382 598 A1

## INTERNATIONAL SEARCH REPORT

| | International application No. |
|---|---|
| | **PCT/JP2022/029522** |

### A. CLASSIFICATION OF SUBJECT MATTER

*C12N 5/074*(2010.01)i; *C12N 5/071*(2010.01)i; *C12N 5/0775*(2010.01)i; *C12N 5/10*(2006.01)i
FI: C12N5/074 ZNA; C12N5/0775; C12N5/10; C12N5/071

According to International Patent Classification (IPC) or to both national classification and IPC

### B. FIELDS SEARCHED

Minimum documentation searched (classification system followed by classification symbols)

C12N5/074; C12N5/071; C12N5/0775; C12N5/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | CN 108849855 A (CHENGDU QINGKE BIO TECH CO LTD) 23 November 2018 (2018-11-23)<br>claims, paragraphs [0002], [0004], [0024], [0037]-[0043], [0072] | 1, 5-14, 16-26, 28-34 |
| X | WO 2018/159797 A1 (FUJIFILM CORP) 07 September 2018 (2018-09-07)<br>paragraphs [0013], [0026], [0042], [0057], [0064], [0071]-[0074] | 1-26, 28-34 |
| Y | | 27 |
| Y | WO 2011/108537 A1 (FUJIFILM CORP) 09 September 2011 (2011-09-09)<br>paragraph [0006], examples 1, 3 | 27 |
| X | CN 108578784 A (QU, Pingbo) 28 September 2018 (2018-09-28)<br>example 1, paragraph [0057] | 26-27 |

☐ Further documents are listed in the continuation of Box C.　　☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **05 October 2022** | **18 October 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

<table>
<tr><td>**INTERNATIONAL SEARCH REPORT**</td><td>International application No.<br>**PCT/JP2022/029522**</td></tr>
</table>

**Box No. I          Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

    The "form of an Annex C/ST.25 text file" is replaced with the "form of ST.26."

Form PCT/ISA/210 (continuation of first sheet) (January 2015)

**INTERNATIONAL SEARCH REPORT**
Information on patent family members

International application No.

**PCT/JP2022/029522**

| Patent document cited in search report | | | Publication date (day/month/year) | Patent family member(s) | Publication date (day/month/year) |
|---|---|---|---|---|---|
| CN | 108849855 | A | 23 November 2018 | (Family: none) | |
| WO | 2018/159797 | A1 | 07 September 2018 | US 2020/0015475 A1 paragraphs [0029], [0046], [0072], [0099], [0111], [0135]-[0143] EP 3590952 A1 | |
| WO | 2011/108537 | A1 | 09 September 2011 | US 2013/0004549 A1 paragraph [0006], examples 1, 3 EP 2543398 A1 CN 102791301 A KR 10-2013-0027480 A | |
| CN | 108578784 | A | 28 September 2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018159797 A
- EP 1014176 A2
- US 6992172 B
- WO 200485473 A
- WO 2008103041 A
- JP 2021126706 A

**Non-patent literature cited in the description**

- Pathophysiology. Nagai Publishing Co., Ltd, 1990, vol. 9, 527
- **MAXEY, C.R.** Phitogr. Gelatin 2. P.J. Academic, 1976
- *J. Mol. Biol.,* 1990, vol. 215, 403-410